# EUROPÄISCHE PATENTANMELDUNG

(11) **EP 0 527 540 A1**
(43) Veröffentlichungstag der Anmeldung: **17.02.1993**
(21) Anmeldenummer: 92250175.4
(22) Anmeldetag: 03.07.1992
(51) Int. Cl.: A61K 31/495, A61K 31/00

(54) **Arzneimittel zur Behandlung von Entzugssymptomen**

(30) Priorität: 09.07.1991 DE 4123106
(71) Anmelder: SCHERING AKTIENGESELLSCHAFT, D-13342 Berlin (DE)
(72) Erfinder: Steppuhn, Karin Gabriele, W-1000 Berlin 41 (DE); Bressler, Karin, W-1000 Berlin 37 (DE); Gieseler, Martin, W-1000 Berlin 48 (DE); Stephens, David Norman, Dr., W-1000 Berlin 19 (DE); Turski, Lechoslaw, Dr., W-1000 Berlin 20 (DE)

(57) **Zusammenfassung**

Die Verwendung von Nicht-NMDA-Rezeptor-Antagonisten oder deren physiologisch verträgliche Salze zur Behandlung von Entzugssymptomen sowie für die Unterdrückung der Abhängigkeit nach Drogenmißbrauch sowie die Kombination der neuen Arzneimittel mit NMDA-Antagonisten wird beschrieben.

## Beschreibung

Die Erfindung betrifft die Verwendung von Nicht-NMDA-Antagonisten oder deren physiologischen Salzen für die Herstellung von Arzneimitteln zur Behandlung von Entzugserscheinungen sowie für die Unterdrückung der Abhängigkeit nach Drogenmißbrauch sowie die Kombination der Arzneimittel mit NMDA-Antagonisten.

Sowohl in der Klinik als auch in der Praxis werden bei Krampfleiden und Schlafstörungen bzw. zur Sedierung und Angstlösung häufig chronische Behandlungen mit benzodiazepinrezeptorbindenden Pharmaka, wie z.B. Diazepam (Valium), durchgeführt. Das größte Problem für die Patienten sind die nach Absetzen dieser Stoffe auftretenden Entzugserscheinungen wie Muskelsteifigkeit, Tremor, Krämpfe und Angstzustände, unter denen die Patienten leiden. Diese Entzugserscheinungen treten auch nach Einnahme von Drogen bzw. Substanzen mit Mißbrauchspotential auf, insbesondere nach Absetzen der Behandlung mit Arzneimitteln, die eine Sucht hervorrufen, wie beispielsweise Benzodiazepinen, Opiaten, Halluzinogenen, Barbituraten oder nach anderen Rauschmitteln wie Alkohol, Kokain oder Heroin. Als Stoffe, die eine physische und psychische Abhängigkeit hervorrufen können, seien die folgenden beispielsmäßig genannt:
1.) Opiate wie Morphin und dessen Derivate, sowie Substanzen mit Morphin-Wirkung wie Methadon, Pethidin und Meperidin sowie Codein und Heroin;
2.) Halluzinogene wie Fentanyl, α-Methylfentanyl, 3-Methylfentanyl, Etryptamin, Dimethyltryptamin und Amphetamine beispielsweise Amphetamin, Methamphetamin, Phenmetrazin, Methylphenidat und Dexamphetamin;
3.) Verbindungen mit sedativen Wirkungen wie Benzodiazepine beispielsweise Diazepam und Chlordiazepoxid, Meprobamat und Barbiturate beispielsweise Hexobarbital, Phenobarbital und Heptabarbital;
4.) Alkohol wie Ethanol und Kokain.

Die Rolle von exzitatorischen Aminosäuren im Zentralnervensystem hat in den letzten Jahren zunehmendes Interesse gewonnen. So wurde Glutamat als Neurotransmitter identifiziert und drei weitere Rezeptorsubtypen für exzitatorische Aminosäuren gefunden und charakterisiert, die nach den spezifisch wirksamen, Glutamat-analogen Aminosäuren N-Methyl-D-Aspartat (NMDA), Kainat- und Quisqualat-Rezeptoren benannt wurden.

Die Benzodiazepine sind die am besten beschriebenen Psychopharmaka, von denen bekannt ist, daß die Langzeitbehandlung erwiesenermaßen zu Toleranz und Abhängigkeit führt. Plötzliche Unterbrechung der Einnahme kann zu unerwünschten Entzugserscheinungen wie Krämpfen, Muskelsteifigkeit und Angstzuständen führen, deren Mechanismen bis jetzt weitgehend ungeklärt sind, da sensitive Methoden zur objektiven Beschreibung der Entzugssymptome im Tierexperiment fehlen.

Der Entzug wird als ein zeitabhängiges aber homogenes klinisches und pathophysiologisches Geschehen betrachtet, dessen Symptome auf die Anwesenheit eines Gamma-Aminobutyrat-BDZ/Cl ionophor Rezeptor-Effektor-Komplexes zurückzuführen ist.

Überraschenderweise wurde gefunden, daß excitatorische Aminosäuren an der Entstehung der Abhängigkeit beteiligt sind und daß die Blockade des Quisqualat-Rezeptors im Entzug die Symptome des Entzugs verhindert oder reduziert.

Gegenstand der Erfindung ist die Verwendung von Nicht-NMDA-Antagonisten oder ihren physiologisch verträglichen Salzen für die Herstellung von Arzneimitteln zur Behandlung von Entzugserscheinungen nach Absetzen der Therapie mit sedierenden Stoffen, Opiaten, Halluzinogenen oder anderen Rauschmitteln wie Kokain oder Heroin oder nach der Einnahme von Alkohol.

Gegenstand der Erfindung ist ferner die Verwendung von Nicht-NMDA-Antagonisten oder ihrer Salze für die Herstellung von Arzneimitteln zur Unterdrückung der Abhängigkeit von Drogen bzw. von Arzneimitteln oder Substanzen mit Mißbrauchspotential.

Die Methoden zur Feststellung der Entzugssymptome basieren auf der Ableitung von elektrischen Potentialen des Gehirns (EEG), der elektromyographischen Ableitung der Muskelaktivität (EMG) und der explorativen Aktivität während des Entzuges bei Mäusen wie am Beispiel der Entzugssymptome nach Benzodiazepinentzug gezeigt wird.

Krämpfe, Muskelspasmen und Angstzustände gehören zu den bekanntesten Entzugserscheinungen, die nach Absetzen einer Langzeittherapie mit BDZ beim Menschen auftreten. Um die Entzugssymptomatik nach chronischer Gabe von Benzodiazepinen zu untersuchen, wurden männliche NMRI Mäuse mit einem Gewicht von 20 - 24 g unter täglichen kontrollierten Verhältnissen (6.00 - 18.00 Uhr Hell/Dunkelrythmus, 45-55 % Luftfeuchte und freiem Zugang zu Wasser und Futter) 12 Tage lang mit 15 mg/kg Diazepam in Sesamöl durch subkutane Injektion tolerant gemacht. Die Kontrolltiere wurden unter gleichen Bedingungen 12 Tage lang mit dem Vehikel s.c. behandelt. Die zwölftägige Behandlung mit 15 mg/kg Diazepam führt bei Mäusen zu einer totalen Toleranz in der sedierenden, antikonvulsiven und anxiolytischen Eigenschaft. Die Aufzeichnung der Entzugssymptome wurde einen Tag nach der letzten subkutanen Injektion gestartet. Dieser Tag wurde als erster Entzugstag benannt. Mit diesem Tag bekommen alle Tiere eine tägliche s.c. Injektion mit dem Vehikel.

Die Aufzeichnung der spontan auftretenden Krämpfe wurde mit Hilfe eines Video- und Computergesteuerten EEG-Aufzeichungssystem vorgenommen. Dieses System erlaubt eine simultane Videoaufnahme von acht Tieren gleichzeitig. Den Tieren wurde zur Ableitung ihres EEGs jeweils eine tiefe hippokampale Elektrode und eine oberflächliche kortikale Elektrode implantiert. Die Aufzeichnungen wurden an 21 aufeinanderfolgenden Tagen mit kurzer täglicher dreißigminütiger Unterbrechung (zwischen 8.30 und 9.00 Uhr) zur Versorgung der Tiere durchgeführt.

Die Untersuchung des sich verändernden Muskeltonus im Entzug wurde mit Hilfe des EMG vom M. gastrocnemius abgeleitet. Hierzu wurden die Mäuse in belüftete Einzelboxen gesetzt. Ihre Hinterextremitäten wurden durch zwei sich im Boden der Boxen befindlichen Löcher gestreckt und vorsichtig mit Klebestreifen befestigt.

Die Untersuchung auf Angstzustände wurde mit Hilfe einer speziell konstruierten Lokomotionslage getestet. Hierzu wurden die Tiere in Einzelboxen gesetzt, die durch Lichtschranken die Bewegungsabläufe der Tiere aufzeichnen. Das sich Hin- und Herbewegen an nur einer Seite der Lokomotionsboxen oder das Verharren in einer Ecke wurde als Maß für Angst benutzt.

Excitatorische Aminosäuren (EAA) besonders die L-Glutamatrezeptor-Subtypen wie N-Methyl-D-aspartat (NMDA), alpha-Amino-3-hydroxy-5-tert-butyl-4-isoxazolpropionat (ATPA) und Kainat (KA) haben die Fähigkeit bei intracerebroventrikulärer Gabe Krämpfe auszulösen. Die Krampfschwelle der excitatorischen Aminosäuren wurde an 21 Tagen im Entzug durch eine intracerebroventrikuläre Infusion an sich frei bewegenden Mäusen getestet. Diese Art der Applikation wurde gewählt, da diese Aminosäuren die Blut-Hirnschranke nur schwer passieren.

Ferner wurden die spinalen Reflexe an 21 Tagen im Diazepamentzug, die unter einer Chloralose/Urethan-Anästhesie abgeleitet wurden, untersucht. Der Hoffmann Reflex (H-) ist ein monosynaptischer Reflex und abhängig von den AMPA-Rezeptoren, die nach den spezifischen Agonisten (RS)-α-Amino-3-hydroxy-5-methyl-4-isoxazolpropionat benannt sind (nicht NMDA-Rezeptoren). Im Gegensatz hierzu sind die Beugereflexe polisynaptisch und NMDA vermittelt. Zur Ableitung des H-Reflexes wurde der N. tibialis ein- bis dreimal mit geregelten Stromstößen stimuliert. Die dabei verwendeten Elektroden wurden oberflächlich am plantaren Fußmuskel angebracht. Die Ableitung der Beugereflexe (Reizschwelle und EMG des Nerven) wurde vom M. tibialis durch fünf elektrische Impulse ein- bis dreimal hintereinander ausgeführt. Hierfür wurde das Elektrodenpaar percutan im ipsilateralen M. tibialis angebracht.

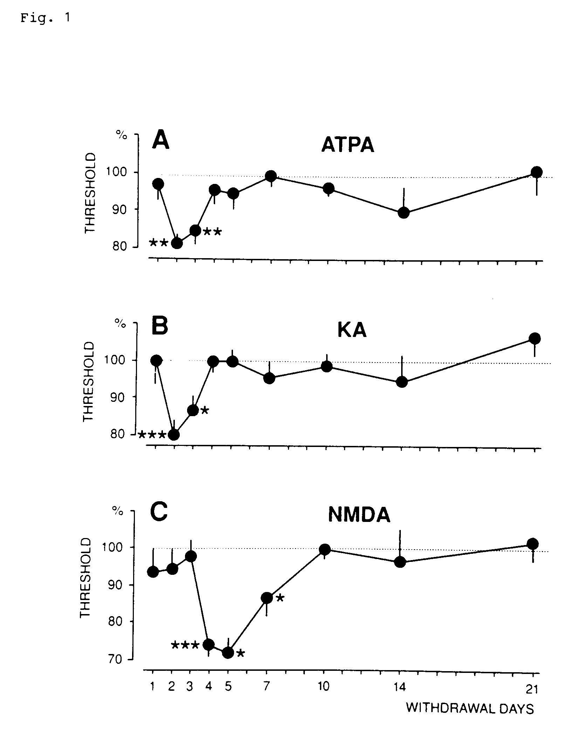

Die Ergebnisse der Tierversuche sind wie folgt zu interpretieren:
Die Krampfschwelle, induziert durch ATPA und KA im Diazepamentzug war am zweiten und dritten Tag erniedrigt und unterschied sich ab dem Tag vier bis zum Tag einundzwanzig nicht mehr von denen der Kontrolltiere. Zur gleichen Zeit an den Tagen zwei und drei blieb die Krampfschwelle für NMDA auf Kontrollniveau. An den Tagen vier bis sieben wurde jedoch eine Erniedrigung der NMDA Krampfschwelle beobachtet, die sich bis zum Tag einundzwanzig jedoch wieder normalisierte (Abb. 1).

Die Ableitung des Hoffmann-Reflexes zeigt einen Anstieg des Schwellenwertes während der ersten drei Tage des Entzuges. Das Maximum des Beugereflexes war jedoch zu einem späteren Zeitpunkt, zwischen den Tagen vier und einundzwanzig.

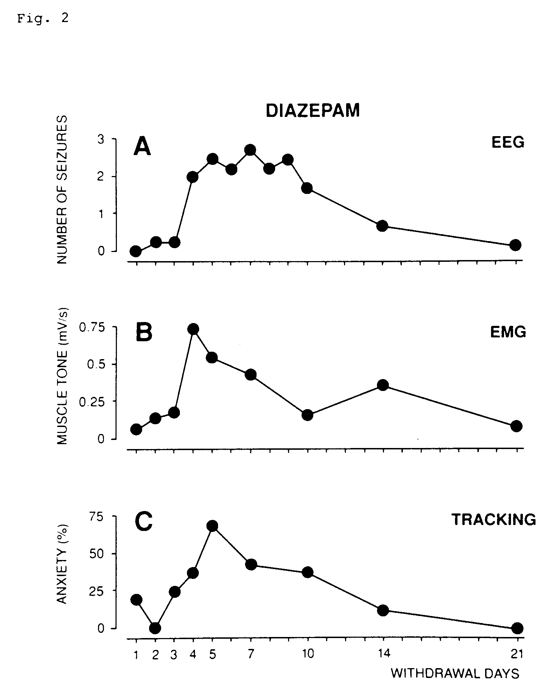

Im EMG wurde eine Steigerung des Muskeltonus ab dem vierten Tag beobachtet, die sich jedoch bis zum Tage einundzwanzig wieder auf Kontrollniveau reduzierte (Abb. 2B).

Bei der Untersuchung auf Angstzustände im Entzug zeigte sich ab dem Tage vier ein verändertes Laufverhalten der Tiere. Sie bewegten sich vorzugsweise in einer Ecke oder liefen nur an einer Wand der Lokomotionsboxen entlang, während die Kontrolltiere des gesamten ihnen zur Verfügung stehenden Raum nutzten und kreuz und quer in den Boxen herumliefen (Abb. 2C).

Die über einundzwanzig Tage dauernde elektroencephalographische Aufzeichnung der Gehirnströme der Tiere läßt ab dem Tage vier Krampfpotentiale erkennen, die erst bis zum Ende des einundzanzigsten Tages wieder abklingen (Abb. 2A).

Diese Daten zeigen, daß der Entzug von Diazepam bei Mäusen mit Hilfe elektrophysiologischer Methoden dargestellt und quantifiziert werden kann. EEG, EMG und die Aktivitätsmessung stellen zuverlässige Methoden dar, um die Entzugssymtome zu erfassen. Die Langzeitableitung im EEG zeigt, daß im Entzug zwei wichtige klinische relevante Phasen unterschieden werden können. Die Krampfschwellenexperimente mit selektiven Antagonisten und die Reflexpharmakologie zeigen, daß die initiale Phase des Diazepamentzuges über den Nicht-NMDA Mechanismus läuft. Diese erste Phase ist sehr kurz und symptomlos doch sicherlich maßgeblich an der Entstehung der zweiten symptomreichen Phase beteiligt. Die erste Phase wird als "silent" und die zweite als "active" bezeichnet.

Aus den Ergebnissen läßt sich erkennen, daß die "silent phase" durch nicht NMDA- und die "active phase" durch NMDA-Mechanismen vermittelt wird.

Um die weitere Rolle der excitatorischen Aminosäuren in der "silent phase" im Entzug von Stoffen mit sedierender Wirkung zu untersuchen, wurden die Tiere mit Quisqualat-Antagonisten oder NMDA-Antagonisten behandelt. Hierbei wurden in der "silent phase" im Diazepamentzug unter einer leichten Äthernarkose osmotisch wirkende Minipumpen intraperitoneal implantiert und die Entzugserscheinungen im EEG, EMG und der Lokomotionsanlage von Tag zwei bis Tag einundzwanzig untersucht. Die Pumpen wurden vorher mit Quisqualat-Antagonisten oder NMDA-Antagonisten gefüllt. Die Pumpenrate betrug über 72 Stunden 10 mg/kg/h.

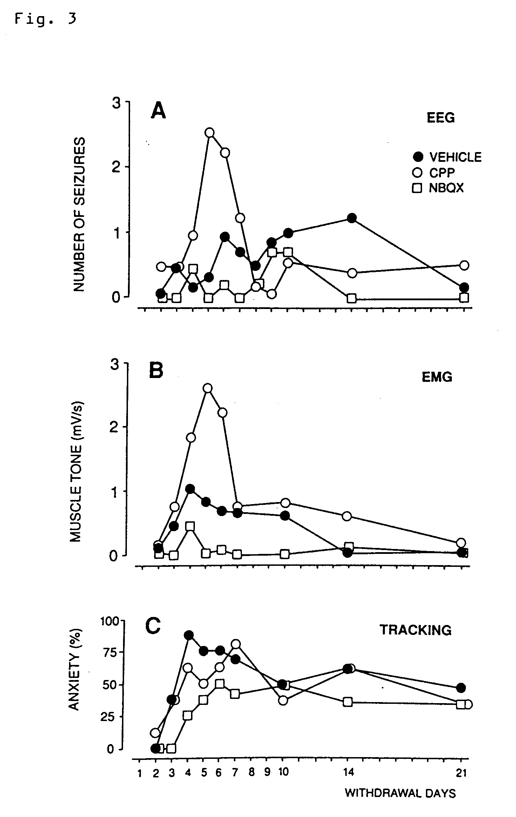

Durch die Behandlung mit Quisqualat-Antagonisten während der ersten 3 Tage des Diazepamentzuges wird die Entstehung von Krämpfen und die Steigerung des Muskeltonus verhindert und die Angst der Tiere reduziert wie am Beispiel der Behandlung mit NBQX gezeigt wird (Abb. 3). Im Gegensatz dazu zeigt die dreitägige Infusion von NMDA-Antagonisten wie CPP (3-((±)2-Carboxy-piperazin-4-yl)-propyl-1-phosponsäure) keine Aufhebung der Entzugssymptome (Abb. 3).

Daß die erste Phase die ausschlaggebende für die klinischen Ergebnisse ist, zeigt der Einsatz des Quisqualat-Antagonisten NBQX in der "silent phase", der die Entstehung der klinischen Symptome vollständig unterbindet und daß im Gegensatz dazu der NMDA-Antagonist CPP keinerlei Einfluß auf die Entstehung der Entzugssymptome hat.

Die Blockade des Nicht-NMDA-Rezeptors in der ersten Phase des Entzuges (silent phase) ist ausreichend, um die Entwicklung der symptomreichen Phase zu reduzieren bzw. zu verhindern.

Diesen Untersuchungen ist zu entnehmen, daß die Behandlung während der ersten Phase des Entzuges mit Nicht-NMDA-Antagonisten die folgenden klinischen Ergebnisse bestimmt und das Entstehen von Entzugserscheinungen verhindert.

Erfindungsgemäß geeignet sind Quisqualat-Antagonisten mit selektiver und nicht-selektiver Wirkung auf AMPA-Rezeptoren, die beispielsweise in EP-A-374534, EP-A-348872, EP-A-283959, EP-A-377112, EP-A-315959, den Dänischen Patentanmeldungen Nos. 1622/91, 1623/91, 1624/91 und 0730/91 und der Deutschen Patentanmeldung P 41 35 871.6 beschrieben sind und nicht kompetitive Quisqualat-Antagonisten wie z.B. GYKI 52466 (1-(4-Aminophenyl)-4-methyl-7,8-methylenedioxy-5H-2,3-benzodiazepin); Tofisopam, Ro 15-1788 (Ethyl-8-fluoro-5,6-dihydro-5-methyl-6-oxo-4H-imidazo(1,5a)(1,4)benzodiazepin-3-carboxylat und die in der Deutschen Patentanmeldung P 42 12 529.4 genannten β-Carboline und Quisqualat-Antagonisten mit selektiver oder nicht-selektiver Wirkung auf metabotrope Rezeptoren wie z.B. L-2-Amino-4-phosphonobuttersäure (AP4) oder L-2-Amino-3-phosphonopropionsäure (AP3) und Kainat-Antagonisten wie γ-Glutamylaminomethylsulfonsäure, sowie deren physiologisch verträgliche Salze, die sich von Alkali-oder Erdalkalimetallen oder den üblichen anorganischen und organischen Säuren ableiten wie Salzsäure, Bromwasserstoffsäure, Schwefelsäure, Phosphorsäure, Zitronensäure, Maleinsäure oder Fumarsäure.

Insbesondere geeignet sind Ouinoxalin-Derivate und deren tautomere Formen mit selektiver und nicht selektiver Wirkung auf AMPA-Rezeptoren.

Bevorzugte Ausführungsformen sind Chinoxalindionderivate und deren tautomere Formen der Formel I 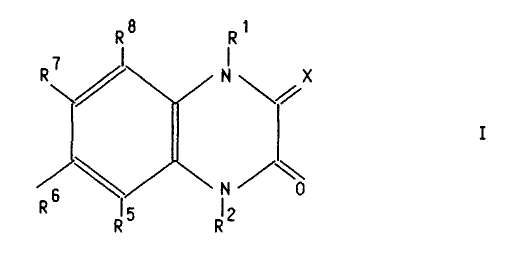
worin
R¹ und R² jeweils Wasserstoff oder einen in den zitierten Patenten genannten Substituenten darstellen und
X Sauerstoff oder gemeinsam mit R¹ die Gruppierung = N-NR³-CO- oder die Gruppierung =N-N=CR³ bedeutet und
R⁵, R⁶, R⁷ und R⁸ gleich oder verschieden sind und jeweils Wasserstoff, NO₂, NH₂, Cyano, Halogen (Fluor, Chlor, Brom oder Jod), CF₃, SO₂NR'R', SO₂R' oder OR' bedeuten und R' Wasserstoff oder C₁₋₄-Alkyl ist oder R⁵ und R⁶ oder R⁷ und R⁸ jeweils gemeinsam einen ankondensierten Benzol-oder Hetarylring oder -(CH₂)₄- bedeuten und R³ Wasserstoff, C₁₋₆-Alkyl oder CF₃ ist, wobei der Benzol- oder Hetarylrest ein-bis dreifach gleich oder unterschiedlich substituiert sein kann mit NO₂, NH₂,
Cyano, Halogen, CF₃, SO₂NR'R', SO₂R' oder OR', wobei R' die obige Bedeutung hat. Geeignete Hetarylringe sind Pyridin, Pyrazol, Thiophen, Pyrazin, Triazol, Imidazol, geeignete Substituenten R¹ und R² sind gegebenenfalls mit Hydroxy, NH₂, Carboxy, Carbonsäureestern, Carbonsäureamiden, Phosphonsäure. Phosphonsäureester oder Phosponsäureamiden substituiertes C₁₋₁₂-Alkyl, C₃₋₈-Cycloalkyl, C₄₋₈-Cycloalkylalkyl, gegebenenfalls mit Phosphonsäure, Phosphonsäureestern oder Phosphonsäureamiden substituiertes C₆₋₁₀-Aryl insbesondere Phenyl, gegebenenfalls mit Phosphonsäure, Phosphonsäureestern oder Phosphonsäureamiden substituiertes C₇₋₁₁-Aralkyl insbesondere Benzyl, C₂₋₇-Alkanoyloxy, Hydroxy, C₁₋₆-Alkoxy, C₆₋₁₀-Aryloxy insbesondere Benzyloxy, C₃₋₈-Cycloalkyloxy und C₄₋₈-Cycloalkylalkyloxy.

Insbesondere bevorzugt sind Chinoxalin- und Benzochinoxalin-Derivate, deren tautomere Formen und Salze, die gegebenenfalls ein- bis zweifach mit NO₂, Halogen, C₁₋₆-Alkyl, Cyano, CF₃, SO₂NR'R', SO₂R' oder OR' substituiert sind, wobei R' Wasserstoff oder C₁₋₄-Alkyl ist und R¹ und R² gleich oder verschieden sind und Wasserstoff oder gegebenenfalls mit -CO-R⁴, -POXY, NR⁷R⁸ oder Phenyl substituiertes C₁₋₁₂-Alkyl bedeuten und X Sauerstoff oder R¹ und X gemeinsam die Gruppierungen =N-NR³-CO-oder =N-N=CR³- bedeuten, wobei R³ Wasserstoff, C₁₋₆-Alkyl oder CF₃ ist und R⁴, X und Y jeweils Hydroxy, C₁₋₆-Alkoxy oder NR⁷R⁸ bedeuten und R⁷ und R⁸ gleich oder verschieden sind und Wasserstoff, C₁₋₆-Alkyl oder gemeinsam mit dem Stickstoffatom einen gesättigten 5- oder sechsgliedrigen Heterocyclus bilden, der ein weiteres O-, N- oder S-Atom enthalten kann wie Piperidin, Pyrrolidin, Morpholin, Thiomorpholin oder Piperazin.

Ganz besonders bevorzugt seien beispielsweise 6-Nitro-7-sulfamoylbenzo-[f]-quinoxalin-2,3-(1H, 4H)-dion (NBQX), 6,7-Dinitroquinoxalin-2,3-dion (DNQX) und 6-Cyano-7-nitroquinoxalin-2,3-dion (CNQX) genannt.

Im Gegensatz zum Quisqualat-Antagonisten NBQX, der in der "silent phase" die Entstehung der klinischen Symptome vollständig unterbindet, hat der NMDA-Antagonist CPP keinerlei Einfluß auf die Entstehung der Entzugssymptome. Bei den Tieren mit CPP Behandlung war die Enzugssymptomatik schneller und ausgeprägter als bei den vergleichbaren Kontrolltieren. Die elektrographisch aufgezeichneten Krämpfe dauerten länger, waren generalisiert und hatten eine höhere Frequenz. Das gleiche zeigte sich im EMG, in dem die Muskelaktivität stark gesteigert war. Die Ergebnisse zeigen, daß die Aktivierung der Nicht-NMDA-Rezeptoren vor den NMDA-Rezeptoren für die Auslosung der Entzugssymptome notwendig ist.

Die vorliegende Erfindung betrifft auch die Verwendung von Nicht-NMDA-Antagonisten in Kombination mit NMDA-Antagonisten zur Behandlung von Entzugserscheinungen und/oder der Unterdrückung der Abhängigkeit nach Drogenmißbrauch. Vorzugsweise wird eine Zweiphasen-Kombination verwendet, wobei in der ersten Phase Nicht-NMDA-Antagonisten und in der anschliessenden Phase NMDA-Antagonisten verabreicht werden.

Als geeignete NMDA-Antagonisten seien beispielmäßig genannt:
**kompetitive Antagonisten** - 2-Amino-7-phosponoheptansäure (AP 7) und Analoga; 3-((+₋)2-Carboxy-piperazin-4-yl)-propyl-1-phosponsäure (CPP) und Analoga; (e)-4-(3-Phosponoprop2-enyl)piperazine-2-carboxylsäure (CPP-ene) und Analoga; S-α-Amino-5-phosphonomethyl-[1,1'-biphenyl]-3-propansäure, E-2-Amino-4-methyl-5-phosphono-3-pentensäure, E-2-Amino-4-methyl-5-phospono-3-pentensäure-ethylester, cis-4-Phoshonomethyl-2-piperidinecarbonsäure, (R)-4-Oxo-2-amino-5-phosphono-pentansäure, 2-Amino-4,5-(1,2-cyclohexyl)-7-phosphonoheptansäure, 4-(Phosphonomethyl)-DL-phenylglycin, 4-(3-Phosphonopropyl)-2-piperidinecarbonsäure, 2-(2-Phosphonoethyl)-DL-phenylalanin, 3-Carboxy-5-(phosphonoethyl)-1,2,3,4-tetrahydroisoquinolin, 3-Carboxy-5-phosphono-1,2,3,4-tetrahydroisoquinolin, cis-DL-4-[(1(2)H-Tetrazol-5-yl)methyl]2-piperidinecarbonsäure, cis-4-(3-Phosphonoprop-1-enyl)-2-piperidinecarbonsäure, E-2-Amino-4-propyl-5-phosphono-3-pentensäure, Phosphorsäure-4-(2-carboxy-piperidinyl)ester und 1-[4(4-chloro-α,α-dimethylbenzyl)-2-methoxyphenyl]-1,2,4-triazole-3-carbonsäureamid;
**Nicht-kompetitive Antagonisten** (+)10,11-Dihyro-5-methyl-5H-dibenzo-[a,d]-cycloheptan-5,10imin (MK-801) und Analoga; Memantine und andere Amantadin-Analoga; Ketamin und Analoga, Budipin und Analoga; Ifenprodil und Analoga; **Antagonisten der Glycinbindungsstelle** - Kynurensäure und Analoga; 1-Hydroxy-3-amino-pyrrolidin-2-on (HA-966) und Analoga; **Polyimine**-Spermin und Spermidin und Analoga; **Hemmer der excitatorischen Aminosäuren-Synthese.**

Kompetitive HMDA-Antagonisten sind als bevorzugt zu betrachten.

Die Erfindung umfaßt auch pharmazeutische Mittel, die die genannten Verbindungen in einer wirksamen Menge enthalten, deren Herstellung sowie die Verwendung der Verbindungen zur Herstellung von Arzneimitteln, zur Behandlung und Prophylaxe der vorstehend genannten Entzugssymptomatik sowie für die Unterdrückung der Abhängigkeit nach Einnahme von Substanzen
mit Mißbrauchspotential. Die Arzneimittel werden nach an sich bekannten Verfahren hergestellt, indem man den Wirkstoff mit geeigneten TrägerHilfs-und/oder Zusatzstoffen in die Form eines pharmazeutischen Präparates bringt, das insbesondere für die enterale oder parenterale Applikation geeignet ist.

Die Applikation kann oral oder sublingual als Feststoff in Form von Kapseln oder Tabletten oder als Flüssigkeit in Form von Losungen, Suspensionen, Elixieren oder Emulsionen oder rektal in Form von Suppositorien oder in Form von gegebenenfalls auch subcutan anwendbaren Injektionslösungen erfolgen. Als Hilfsstoffe für die gewünschte Arzneimittelformulierung sind die dem Fachmann bekannten inerten organischen und anorganischen Trägermaterialien geeignet wie zum Beispiel Wasser, Gelatine, Gummi arabicum, Milchzucker, Stärke, Magnesiumstearat, Talk, pflanzliche Öle, Polyalkvlenglykole usw. Gegebenenfalls können darüber hinaus Konservierungs-, Stabilisierungs-, Netzmittel, Emulgatoren oder Salze zur Veränderung des osmotischen Druckes oder Puffer enthalten sein.

Die pharmazeutischen Präparate können in fester Form zum Beispiel als Tabletten, Dragees, Suppositorien, Kapseln oder in flüssiger Form, zum Beispiel als Lösungen, Suspensionen oder Emulsionen vorliegen oder als Depotzubereitung formuliert sein.

Als Trägersysteme können auch grenzflächennahe Hilfsstoffe wie Salze der Gallensäuren oder tierische oder pflanzliche Phospholipide, aber auch Mischungen davon sowie Liposome oder deren Bestandteile verwendet werden.

Für die orale Anwendung sind insbesondere Tabletten, Dragees oder Kapseln mit Talkum und/oder Kohlenwasserstoffträger oder -binder, wie zum Beispiel Lactose, Mais- oder Kartoffelstärke, geeignet. Die Anwendung kann auch in flüssiger Form erfolgen, wie zum Beispiel als Saft, dem gegebenenfalls ein Süßstoff beigefügt wird.

Die Dosierung der Wirkstoffe kann je nach Verabfolgungsweg, Alter und Gewicht des Patienten, Art und Schwere der zu behandelnden Erkrankung und ähnlichen Faktoren variieren. Die tägliche Dosis beträgt 0,001 - 0,034 mg, wobei die Dosis als einmal zu verabreichende Einzeldosis oder unterteilt in 2 oder mehrere Tagesdosen gegeben werden kann.

In den erfindungsgemäßen Kombinationspräparaten können die Wirkstoffe in einer Formulierung oder auch in jeweils getrennten Formulierungen vorliegen, wobei die gesamte Dosis einmalig verabreicht oder in mehrere Dosen geteilt wird.

## Patentansprüche

1. Verwendung von Nicht-NMDA-Antagonisten oder deren physiologisch verträgliche Salze für die Herstellung von Arzneimitteln zur Behandlung und Prophylaxe von Entzugssymptomen nach Drogenmißbrauch.

2. Verwendung nach Anspruch 1, dadurch gekennzeichnet, daß die Behandlung sofort nach dem Entzug einsetzt.

3. Verwendung nach Anspruch 1 oder 2, dadurch gekennzeichnet, daß Entzugssymptome nach Einnahme von sedierender Wirkstoffe behandelt werden.

4. Verwendung von Nicht-NMDA-Antagonisten oder deren physiologisch verträgliche Salze für die Herstellung von Arzneimitteln für die Unterdrückung der Abhängigkeit von Drogen.

5. Verwendung nach Anspruch 1-4, dadurch gekennzeichnet, daß der Nicht-NMDA-Antagonist ein Quisqualat-Antagonist, mit selektiver und nicht selektiver Wirkung auf AMPA-Rezeptoren, ein nicht kompetitiver Quisqualat-Antagonist oder ein Quisqualat-Antagonist mit selektiver oder nicht selektiver Wirkung auf metabotrope Rezeptoren ist.

6. Verwendung nach Anspruch 5, dadurch gekennzeichnet, daß der Nicht-NMDA-Antagonist ein Quisqualat-Antagonist mit selektiver und nicht-selektiver Wirkung auf AMPA-Rezeptoren ist.

7. Pharmazeutisches Präparat zur Verwendung nach Anspruch 1 - 6 enthaltend einen Nicht-NMDA-Antagonisten oder deren physiologisch verträgliche Salze.

8. Pharmazeutisches Präparat nach Anspruch 7 in Kombination mit NMDA-Antagonisten oder deren physiologisch verträglichen Salzen.

9. Pharmazeutisches Präparat nach Anspruch 7 und 8 dadurch gekennzeichnet, daß mit einem kompetitiven NMDA-Antagonisten kombiniert wird.
